# EUROPEAN PATENT APPLICATION

(11) **EP 4 763 569 A1**
(43) Date of publication of application: **24.06.2026**
(21) Application number: 25223947.0
(22) Date of filing: 16.12.2025
(51) Int. Cl.: B60K 28/06, B60W 50/14

(54) **DRIVING SUPPORT CONTROL DEVICE, DRIVING SUPPORT METHOD, AND COMPUTER PROGRAM**

(30) Priority: 19.12.2024 JP 2024224256
(71) Applicant: Toyota Jidosha Kabushiki Kaisha, Toyota-shi, Aichi-ken 471-8571 (JP)
(72) Inventor: TANAKA, Yusuke, TOYOTA-SHI, AICHI-KEN, 471-8571 (JP); FUKAYA, Motohiro, TOYOTA-SHI, AICHI-KEN, 471-8571 (JP); TOSHIMA, Kohei, TOYOTA-SHI, AICHI-KEN, 471-8571 (JP); MORIMOTO, Kazuhiro, TOYOTA-SHI, AICHI-KEN, 471-8571 (JP)
(74) Representative: Cabinet Beau de Loménie

(57) **Abstract**

The driving support control device (6) for a vehicle (100) is configured to execute driving support for responding to driver abnormality in response to it being judged that a driver of the vehicle (100) is in an abnormal state in which it is difficult to continue driving of the vehicle (100), and suspend the driving support when a cancel operation is detected a plurality of times during the driving support. The cancel operation includes a pedal operation of a pedal of the vehicle (100), and a predetermined operation other than the pedal operation, and when the pedal operation is not detected even once as the cancel operation, the driving support control device (6) is configured not to suspend the driving support even when the predetermined operation other than the pedal operation is detected the plurality of times.

## Description

### TECHNICAL FIELD

The present invention relates to a driving support control device, a driving support method, and a computer program.

### BACKGROUND ART

Patent Literature 1 discloses a conventional vehicle driving control device which is configured to judge whether a driver is in an abnormal state, and execute deceleration and stop control decelerating the vehicle and maintaining the vehicle in a stopped state, when the driver is in the abnormal state.

### CITATION LIST

### PATENT LITERATURE

PTL 1 Japanese Unexamined Patent Publication (Kokai) No. 2021-109559

### SUMMARY

### [TECHNICAL PROBLEM]

The vehicle driving control device of the prior art described above is configured to judge that the driver is in a non-operation state in which the driver does not perform any driving operations when none of the accelerator pedal operation amount, brake pedal operation amount, and steering torque has changed, and judge that the driver is in an abnormal state when the non-operation state continues for a predetermined time or more. The control device is further configured to judge that driver intervention in the driving operation (hereinafter referred to as "override") has been performed, and suspend the deceleration and stop control when there is a change in any of the accelerator pedal operation amount, the brake pedal operation amount, or the steering torque during the deceleration and stop control.

However, during the deceleration and stop control, there is a possibility that a driver who has fallen into an abnormal state such as a tense, stiff, or relaxed state, etc., may perform an unintentional pedal operation or steering operation. Thus, if the deceleration and stop control is suspended when any of the accelerator pedal operation amount, brake pedal operation amount, or steering torque changes once, in the manner of the case of the vehicle driving control device of the prior art described above, there is a risk that the deceleration and stop control will be suspended due to an unintentional override even though the driver is in an abnormal state.

The present invention has been conceived in light of such problems, and an object thereof is to prevent an unintentional override from causing suspension of driving support for responding to driver abnormality regardless of the driver being in an abnormal state.

### SOLUTION TO PROBLEM

In order to solve the above problem, the driving support control device for a vehicle according to one aspect of the present invention is configured to execute driving support for responding to driver abnormality in response to it being judged that a driver of the vehicle is in an abnormal state in which it is difficult to continue driving of the vehicle, suspend the driving support when a cancel operation is detected a plurality of times during the driving support, and when the cancel operation includes a pedal operation of a pedal of the vehicle and a predetermined operation other than the pedal operation, and the pedal operation is not detected even once as the cancel operation, not suspend the driving support even when the predetermined operation other than the pedal operation is detected the plurality of times.

Further, the driving support method according to one aspect of the present invention is performed by a control device of a vehicle, and includes executing driving support for responding to driver abnormality in response to it being judged that a driver of the vehicle is in an abnormal state in which it is difficult to continue driving of the vehicle, suspending the driving support when a cancel operation is detected a plurality of times during the driving support, and when the cancel operation includes a pedal operation of a pedal of the vehicle and a predetermined operation other than the pedal operation, and the pedal operation is not detected even once as the cancel operation, not suspending the driving support even when the predetermined operation other than the pedal operation is detected the plurality of times.

Further, the computer program according to one aspect of the present invention causes a computer to execute processing of executing driving support for responding to driver abnormality in response to it being judged that a driver of the vehicle is in an abnormal state in which it is difficult to continue driving of the vehicle, suspending the driving support when a cancel operation is detected a plurality of times during the driving support, and when the cancel operation includes a pedal operation of a pedal of the vehicle, and a predetermined operation other than the pedal operation, and the pedal operation is not detected even once as the cancel operation, not suspending the driving support even when the predetermined operation other than the pedal operation is detected the plurality of times.

### ADVANTAGEOUS EFFECTS OF INVENTION

According to these aspects of the present invention, it is possible to prevent an unintentional override from causing suspension of driving support for responding to driver abnormality regardless of the driver being in an abnormal state.

### BRIEF DESCRIPTION OF DRAWINGS

FIG. 1 is a schematic view of a vehicle according to an embodiment of the present invention.
FIG. 2 is a flowchart detailing suspension processing for driving support for driver abnormality according to an embodiment of the present invention.

### DESCRIPTION OF EMBODIMENTS

Embodiments of the present invention will be described in detail below with reference to the drawings. Note that in the following description, identical constituent elements have been assigned the same reference signs.

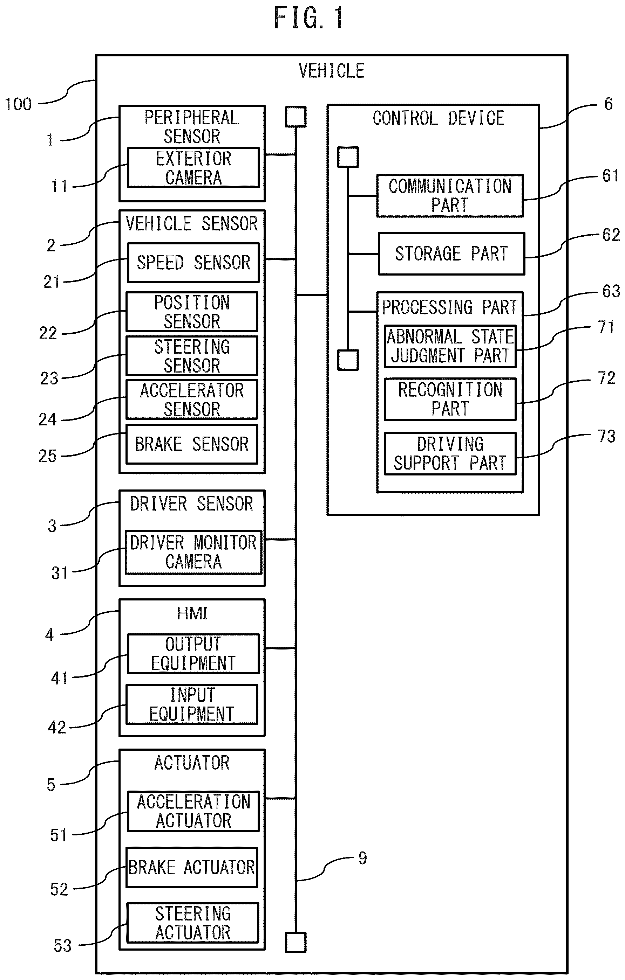

FIG. 1 is a schematic view of a vehicle 100 according to an embodiment of the present invention.

The vehicle 100 includes a peripheral sensor 1, a vehicle sensor 2, a driver sensor 3, a human machine interface (HMI) 4, an actuator 5, and a control device 6. The peripheral sensor 1, the vehicle sensor 2, the driver sensor 3, the HMI 4, the actuator 5, and the control device 6 are communicatively connected to each other via an in-vehicle network 9 which complies with a standard such as controller area network.

The peripheral sensor 1 is a sensor for generating peripheral data representing the situation around the vehicle 100. The vehicle 100 according to the present embodiment includes, as the peripheral sensor 1, one or a plurality of exterior cameras 11 for capturing the surroundings of the vehicle 100. The exterior camera 11 captures the surroundings of the vehicle 100 at a predetermined frame rate (for example, 10 [Hz] to 40 [Hz]) and generates a surrounding image showing the surroundings of the vehicle 100. The exterior camera 11 transmits the generated surrounding image to the control device 6 as surrounding data every time it generates a surrounding image.

In addition to the exterior camera 11, a ranging sensor for measuring the distance to a target or a feature present around the vehicle 100 may be provided as the peripheral sensor 1. Examples of the ranging sensor include LiDAR (Light Detection And Ranging) which irradiates radar light and measures distance based on the reflected light, and a millimeter wave radar sensor which irradiates radio waves and measures distance based on the reflected waves.

The vehicle sensor 2 is a sensor for acquiring vehicle data representing the state of the vehicle 100. The vehicle 100 according to the present embodiment includes, as the vehicle sensor 2, a speed sensor 21 for acquiring speed data representing the traveling speed of the vehicle 100, a position sensor 22 for acquiring current position data representing the current position of the vehicle 100 such as latitude and longitude, a steering sensor 23 for acquiring data related to steering operations including at least one of steering grip, steering torque, and steering angle, an accelerator sensor 24 for acquiring data related to accelerator operation such as the amount of depression of the accelerator pedal, and a brake sensor 25 for acquiring data related to brake operation such as detection of operation input of the brake pedal. However, the vehicle sensor 2 is not limited to these sensors. Each type of data acquired by each sensor 21 to 25 is transmitted to the control device 6 as vehicle data.

The driver sensor 3 is a sensor for generating driver data representing the state of the driver. The vehicle 100 according to the present embodiment includes, as the driver sensor 3, a driver monitor camera 31 for capturing the appearance of the driver including the face of the driver. The driver monitor camera 31 captures the appearance of the driver at a predetermined frame rate (for example, 10 [Hz] to 40 [Hz]) and generates an appearance image showing the appearance of the driver. The driver monitor camera 31 transmits the generated appearance image to the control device 6 as driver data every time it generates an appearance image of the driver.

The HMI 4 is a user interface for exchanging information between the vehicle 100 and the occupants thereof. The HMI 4 includes output equipment 41 for notifying the vehicle occupants via the bodily senses (for example, vision, hearing, touch, etc.) of the vehicle occupants, and input equipment 42 with which the vehicle occupants can perform input operations and response operations. The output equipment 41 is, for example, a display (for example, a meter display, a center display, a heads-up display, etc.) and a speaker. The input equipment 42 is, for example, a touch panel and a microphone.

The HMI 4 notifies the vehicle occupants of information corresponding to the output signal received from the control device 6 via the output equipment 41, and transmits data input by the vehicle occupants to the control device 6 via the input equipment 42.

The HMI 4 may be installed in advance in the vehicle 100, or may be a terminal such as a smartphone owned by the vehicle occupants (driver and passengers). In the latter case, for example, information may be exchanged between the vehicle 100 and the terminal of the vehicle occupants by short-range wireless communication, or information may be exchanged indirectly via the server by communicating between the terminal of the vehicle occupants and an external server (not illustrated).

The actuator 5 is a device used for driving control of the vehicle 100. The vehicle 100 according to the present embodiment includes, as the actuator 5, an acceleration actuator 51 (for example, at least one of an engine and a motor) for performing acceleration control of the vehicle 100, a brake actuator 52 (for example, a hydraulic actuator) for performing brake control of the vehicle 100, and a steering actuator 53 (for example, a steering motor) for performing steering control of the vehicle 100.

The control device 6 is an electronic control unit (ECU) including a communication part 61, a storage part 62, and a processing part 63.

The communication part 61 includes an interface circuit for connecting the control device 6 to the in-vehicle network 9. The communication part 61 supplies various data received from the outside to the processing part 63. The communication part 61 also outputs various signals output from the processing part 63 to the outside.

The storage part 62 includes a storage medium such as a hard disk drive (HDD), a solid disk drive (SSD), or a semiconductor memory, and stores various computer programs and data used in the processing by the processing part 63.

The processing part 63 includes one or more central processing units (CPUs) and peripheral circuits therefor, and executes various computer programs stored in the storage part 62. The processing part 63 is, for example, a processor. The processing part 63 may further include other arithmetic circuits such as a logic arithmetic unit, a numerical arithmetic unit, or a graphic processing unit. The processing part 63 executes processing in accordance with the computer programs, thereby functioning as an abnormal state judgment part 71, a recognition part 72, and a driving support part 73, and operates as a functional part (module) for realizing predetermined functions. In the following description, when processing in which the functional parts 71 to 73 are the subject is described, it indicates that the processing part 63 is executing a program for realizing each of the functional parts 71 to 73.

Specific processing executed by the control device 6 will be described below. Specifically, the contents of each of the functional parts 71 to 73 realized by the processing part 63 executing the processing according to the computer program will be described.

The abnormal state judgment part 71 judges whether the driver has entered into an abnormal state (hereinafter simply referred to as an "abnormal state") in which it is difficult to continue driving due to, for example, a sudden change in physical condition, etc. In the present embodiment, the abnormal state judgment part 71 judges that the driver has entered into an abnormal state when a predetermined abnormality presumption state continues for a predetermined judgment time T1 [s].

The abnormality presumption state is a state in which the driver is considered to be in an abnormal state. Examples of abnormality presumption states include a state in which the driver has their eyes closed, a state in which the posture of the driver is distorted, and a state in which the driver is not operating the steering wheel unless the driving support that allows hands-free driving is being executed. A state in which the posture of the driver is distorted is, for example, a state in which the driver lies face down, looks down, or leans back due to muscle relaxation caused by loss of consciousness, or the head or upper body of the driver is tilted or fallen to the side, or a state in which the driver is arched due to rigidity caused by epilepsy, etc.

Whether the driver has their eyes closed, the posture of the driver is distorted, or the steering wheel is not being operated can be judged from, for example, the appearance of the driver based on the image of the driver monitor camera 31. Furthermore, whether the steering wheel is not being operated can be judged based on, for example, when data related to the steering operation is acquired by the steering sensor 23, such data and the image of the driver monitor camera 31.

The recognition part 72 recognizes targets and features around the vehicle 100. The recognition part 72 sequentially inputs the surrounding images received from the exterior camera 11 to a classifier, for example, to recognize targets in the surrounding images, such as other vehicles, motorcycles, and pedestrians, and features such as curbs, fences, and other similar structures (hereinafter referred to as "dividing features") and road markings (for example, dividing lines that define driving lanes). The classifier can be, for example, a convolutional neural network (CNN) having multiple convolution layers connected in series from the input side to the output side. The recognition part 72 also calculates the distances from the vehicle 100 to the targets and features using, for example, the standard sizes of the targets and features stored in the storage part 62 for each type of target and feature and the sizes of the targets and features recognized in the surrounding images, and calculates the positions of the targets and features. Note that the method of recognizing the targets and features is not limited to this method, and various known methods may be used for recognition.

The driving support part 73 controls the actuator 5 based on the targets and features recognized by the recognition part 72, and executes driving support involving driving control of the vehicle 100. In the present embodiment, the driving support part 73 can execute driving support involving driving control of the vehicle 100 at a driving control level of level 3 defined by the Society of Automotive Engineers (SAE), and specifically, a driving control level that does not require the driver to operate each of the actuators 51 to 53 and monitor the surroundings. The driving support part 73 can also execute driving support involving driving control of the vehicle 100 at a driving control level where the driver is involved in driving the vehicle 100, for example, a driving control level of level 1 or level 2 as defined by the SAE.

As one of the driving supports accompanying the driving control of the vehicle 100, when the driver is judged to be in an abnormal state, the driving support part 73 executes driving support for driver abnormality to respond to the driver abnormality. Specifically, when it is judged that the driver is in an abnormal state, the driving support part 73 first executes notification control to notify the driver of a control notice, alarm, or the like via the HMI 4. Thereafter, after a predetermined time T2 [s] has elapsed since starting the notification, the driving support part 73 executes deceleration and stop control (deceleration control and stop maintenance control) to decelerate the vehicle 100 and maintain the vehicle 100 in a stopped state. Specifically, the driving support for driver abnormality includes the notification control and the deceleration and stop control.

Furthermore, how the vehicle 100 is decelerated and stopped is not particularly limited as long as the risk of contact with road users outside the vehicle when executing the deceleration and stop control is taken into consideration. For example, when the dividing line of the lane of the vehicle can be recognized, the vehicle 100 is decelerated along the dividing line of the lane of the vehicle and maintained in the stopped state, when the dividing line of the lane of the vehicle cannot be recognized, the vehicle 100 is decelerated along the trajectory of the vehicle ahead and maintained in the stopped state, and when neither the dividing line of the lane of the vehicle nor the vehicle ahead can be recognized, the vehicle 100 can be decelerated while moving straight and maintained in the stopped state. Furthermore, for example, in consideration of the rescue of the driver and the safety of disembarkation of passengers, the lane may be changed to move the vehicle to the lane on the roadway side or to the shoulder.

Note that when the driver performs an override operation, such as steering, accelerating, or braking, during driving support for driver abnormality, it is desirable to suspend the driving support, since it can be determined that the state of the driver is a normal state in which driving can be continued.

However, during driving support for driver abnormality, for example, a driver who has lost consciousness and fallen face down on the steering wheel may perform an unintentional steering operation, or a driver who has fallen into a rigid state due to epilepsy may perform an unintentional pedal operation. Specifically, during driving support for driver abnormality, there is a risk that an unintentional steering or pedal operation may be performed by a driver who has fallen into an abnormal state. In such a case, there is a risk that the driving support for driver abnormality may be suspended due to an unintentional override, even though the driver is in an abnormal state.

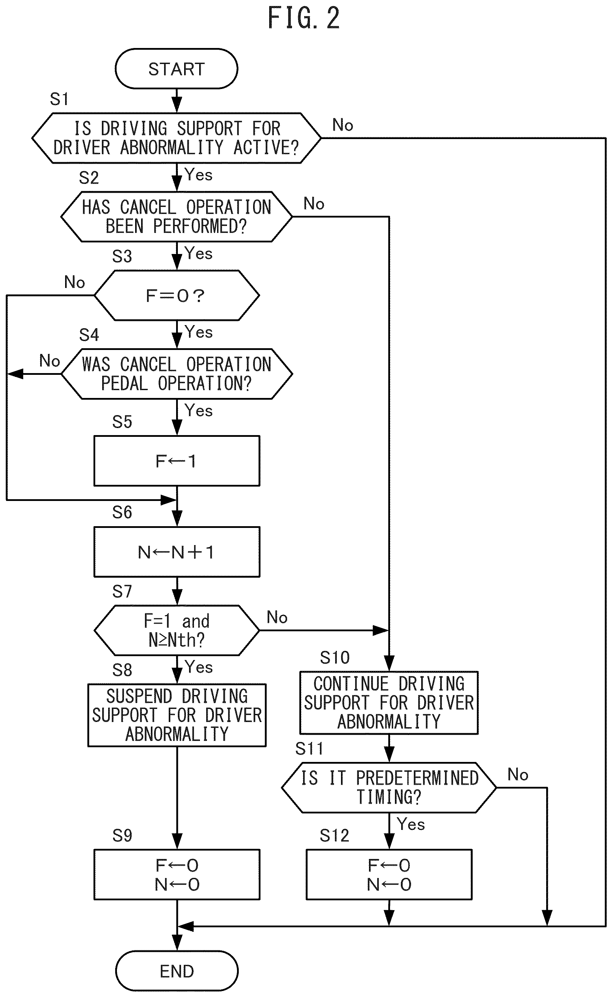

Thus, in the present embodiment, even if an unintentional override is accidentally performed once during the driving support for driver abnormality, the driving support is not suspended. The suspension process of the driving support for driver abnormality according to the present embodiment will be described below with reference to FIG. 2.

FIG. 2 is a flowchart detailing the processing for suspending the driving support for driver abnormality according to the present embodiment, which is executed by the driving support part 73, and in turn, the control device 6. The control device 6 repeatedly executes this routine at a predetermined calculation cycle.

In step S1, the control device 6 judges whether the vehicle is in driving support for driver abnormality. When the vehicle is in driving support for driver abnormality, the control device 6 proceeds to the processing of step S2. Conversely, when the vehicle is not in driving support for driver abnormality, the control device 6 ends the current processing.

In step S2, the control device 6 judges whether a cancel operation has been performed. In the present embodiment, the control device 6 judges that a cancel operation has been performed once each time a steering operation, an accelerator operation, or a brake operation is performed. With regard to whether each operation of a steering operation, an accelerator operation, and a brake operation has been performed, it is possible to judge that the operation has been performed, for example, when the operation change amount of each operation becomes equal to or greater than a predetermined amount, or simply when the operation input of each operation is detected.

Note that accelerator operation and brake operation, i.e., pedal operations, include a depressing operation and a releasing operation. In the present embodiment, regarding the pedal releasing operation among pedal operations, only the operation of releasing the pedal that was depressed at the start of the driving support for driver abnormality is detected as a cancel operation, and the reason for which will be described later.

In step S3, the control device 6 judges whether a pedal operation detection flag F is 0. The pedal operation detection flag F is a flag which is set to 1 when a pedal operation, i.e., an accelerator operation or a brake operation, is performed once during driving support for driver abnormality, and the initial value thereof is set to 0. When the pedal operation detection flag F is 0, the control device 6 proceeds to the processing of step S4. Conversely, when the pedal operation detection flag F is 1, the control device 6 proceeds to the processing of step S6.

In step S4, the control device 6 judges whether the performed cancel operation was a pedal operation. When the performed cancel operation was a pedal operation, the control device 6 proceeds to the processing of step S5. Conversely, when the performed cancel operation was not a pedal operation, the control device 6 proceeds to the processing of step S6.

In step S5, the control device 6 sets the pedal operation detection flag F to 1.

In step S6, the control device 6 increments a count value N of the detection number of the cancel operation by 1. The initial value of the count value N is zero.

In step S7, the control device 6 judges whether the pedal operation detection flag F is set to 1 (i.e., a pedal operation has been performed at least once during the driving support for driver abnormality) and the count value N of the detection number of the cancel operation is equal to or greater than a predetermined driving support suspension threshold Nth. The driving support suspension threshold Nth can be set to any value equal to or greater than 2. In the present embodiment, the driving support suspension threshold Nth is 2. When the pedal operation detection flag F is set to 1 and the count value N of the cancel operation is equal to or greater than the driving support suspension threshold Nth, the control device 6 proceeds to the processing of step S8. Conversely, when the pedal operation detection flag F is 0 or the count value N of the cancel operation is less than the driving support suspension threshold Nth, the control device 6 proceeds to the processing of step S9.

In step S8, the control device 6 suspends the driving support for driver abnormality. Thus, in the present embodiment, at least two or more cancel operations and at least one pedal operation are required to suspend the driving support for driver abnormality.

By requiring the implementation of a cancel operation at least twice in order to suspend the driving support for driver abnormality, the driving support for driver abnormality will not be suspended even if an unintentional override (cancel operation) is performed once by accident. Thus, it is possible to prevent suspension of the driving support for driver abnormality due to an unintentional override even though the driver is in an abnormal state.

Furthermore, for example, when the driver falls into an abnormal state due to muscle relaxation caused by loss of consciousness, the driver may fall face down on the steering wheel, or their upper body may tilt sideways or fall while in a face down state, which may lead to unintentional steering operation. The driver may also fall into an abnormal state while grasping the steering wheel, which may also lead to an unintentional steering operation. In response to this, by requiring at least one pedal operation to be performed for suspending the driving support for driver abnormality, since the driving support for driver abnormality will not be suspended unless a pedal operation is performed, suspension of the driving support for driver abnormality due to an unintentional steering operation can be prevented.

In step S9, the control device 6 resets the pedal operation detection flag F to 0, and also resets the cancel operation count value N to the initial value of zero.

In step S10, the control device 6 continues the driving support for driver abnormality.

In step S11, the control device 6 judges whether it is a predetermined timing during the driving support for driver abnormality. In the present embodiment, the predetermined timing is set to a timing when a predetermined time has elapsed since the notification control started, but is not limited thereto, and can be set to a timing when a predetermined distance has been traveled since the notification control was started, a timing when the notification control is ended, a timing when the deceleration control is started, etc. When it is judged that it is the predetermined timing during the driving support for driver abnormality, the control device 6 proceeds to the processing of step S12. Conversely, when it is judged that it is not the predetermined timing during the driving support for driver abnormality, the control device 6 ends the current processing.

In step S12, the control device 6 resets the pedal operation detection flag F to 0 and resets the count value N of the cancel operation to zero. When the driver is in a normal state but is mistakenly judged to be in an abnormal state and driving support for driver abnormality is started, a normal driver usually performs a plurality of cancel operations at relatively short intervals during the driving support for driver abnormality, and rarely performs cancel operations at relatively long intervals in the early and late stages of the driving support for driver abnormality. Thus, cancel operations performed at relatively long intervals in the early and late stages of the driving support for driver abnormality may be unintentional cancel operations.

Therefore, by returning the count value N to zero once at a predetermined timing during the driving support for driver abnormality, it is possible to prevent the occurrence of a situation in which, for example, an unintentional cancel operation is accidentally performed at the early and late stages of the driving support, causing the driving support to be suspended even though the driver is in an abnormal state.

The control device 6 (driving support control device) of the vehicle 100 according to the present embodiment described above is configured to execute driving support for driver abnormality for responding to abnormality of the driver in response to the driver of the vehicle 100 being judged to be in an abnormal state in which it is difficult to continue driving of the vehicle 100, and suspend the driving support when a cancel operation is detected a plurality of times during the driving support for driver abnormality, and the cancel operation includes a pedal operation of the pedal of the vehicle 100 and a predetermined operation other than the pedal operation, and when the pedal operation is not detected even once as the cancel operation, the control device 6 is configured not to suspend the driving support for driver abnormality even when the predetermined operation other than the pedal operation is detected a plurality of times.

In this manner, by requesting the execution of a cancel operation a plurality of times to suspend the driving support for driver abnormality, the driving support for driver abnormality will not be suspended even if an unintentional override (cancel operation) is performed once by accident. Thus, it is possible to prevent suspension of the driving support for driver abnormality due to an unintentional override even though the driver is in an abnormal state.

As described above, when the driver falls into an abnormal state due to muscle relaxation caused by loss of consciousness, the driver may tend to perform an unintentional steering operation by leaning face down on the steering wheel or tilting or falling to the side while in a prone state. Furthermore, it is possible that the driver may fall into an abnormal state while grasping the steering wheel, and in this case, an unintentional steering operation is also likely to be performed. In response to this, by requiring the execution of at least one pedal operation in addition to the execution of a cancel operation a plurality of times to suspend the driving support for driver abnormality, since the driving support for driver abnormality will not be suspended unless a pedal operation is performed, the driving support for driver abnormality is prevented from being suspended by a steering operation that is relatively likely to be performed as an unintentional cancel operation. Thus, it is possible to more effectively prevent suspension of the driving support for driver abnormality due to an unintentional override even though the driver is in an abnormal state.

Furthermore, the control device 6 of the present embodiment is configured to, when detecting an operation of releasing the pedal as the pedal operation, detect only the operation of releasing the pedal that was depressed at the start of driving support for driver abnormality, as a cancel operation.

When executing the cancel operation a plurality of times is requested to suspend the driving support for driver abnormality in the manner of the present embodiment, when the driver is mistakenly judged as being in an abnormal state despite being in a normal state and the driving support for driver abnormality is executed, the driver will not be able to suspend the driving support for driver abnormality with a single cancel operation, making it difficult to suspend the driving support for driver abnormality.

In this regard, since pedal operations include a depressing operation and a releasing operation, by detecting the releasing operation as one cancel operation, it becomes easier to detect the cancel operation a plurality of times, and thus, suspension of driving support for driver abnormality can be prevented from becoming too difficult when the driver is in a normal state.

However, when the operation of releasing the pedal is detected as one cancel operation, the operation of momentarily depressing and releasing the pedal will be detected as two cancel operations. It is preferable to detect such an operation of momentarily depressing and releasing the pedal as one operation rather than detecting it as a plurality of operations. When it is mistakenly judged that the driver is in an abnormal state even though the driver is in a normal state and driving support for driver abnormality is executed, it is highly likely that the driver is depressing the pedal at the start of the driving support. Thus, by detecting only the operation of releasing the pedal that was depressed at the start of the driving support for driver abnormality as one cancel operation, it is possible to prevent the operation of the driver momentarily depressing and releasing the pedal after the driving support for driver abnormality is started from being detected as two cancel operations.

Accordingly, in the manner of the present embodiment, when an operation of releasing the pedal is detected as the pedal operation, only the operation of releasing the pedal that was pressed at the start of driving support for driver abnormality is detected as a cancel operation, thereby preventing it from becoming too difficult to suspend the driving support for driver abnormality when the driver is in a normal state, while preventing the operation of momentarily pressing and releasing the pedal from being detected as a plurality of operations (two cancel operations).

Furthermore, the control device 6 according to the present embodiment is configured to judge that a cancel operation has been performed once each time either the steering operation or the pedal operation of the vehicle 100 is performed once, and increment the count value N of the detection number of the cancel operation by one.

As a result, when a plurality of types of operations, such as a steering operation and a pedal operation, are accepted as cancel operations, each operation can be counted as one cancel operation.

The control device 6 according to the present embodiment is configured to return the count value N of the detection number of the cancel operation to zero at a predetermined timing during the driving support for driver abnormality. The predetermined timing is, for example, the timing when a predetermined time has elapsed since the notification control started, the time when a predetermined distance has been traveled since the notification control started, the timing when notification control ended, or the timing when the deceleration and stop control started.

As described above, when the driver is in a normal state but is mistakenly judged to be in an abnormal state and driving support for driver abnormality is started, a normal driver usually performs a cancel operation a plurality of times at relatively short intervals during the driving support for driver abnormality, and rarely performs a cancel operation at relatively long intervals in the early and late stages of the driving support for driver abnormality. Thus, cancel operations performed at a relatively long interval in the early and late stages of the driving support for driver abnormality may be an unintentional cancel operation. Therefore, by returning the count value N of the cancel operation to zero at a predetermined timing during the driving support, it is possible to prevent a situation in which an unintentional cancel operation is accidentally performed in the early and late stages of the driving support for driver abnormality and the driving support is suspended even though the driver is in an abnormal state.

Though the embodiments of the present invention have been described above, the embodiments described above merely illustrate some of the application examples of the present invention, and are not intended that the technical scope of the present invention be limited to the specific configurations of the embodiments described above.

Furthermore, for example, in the embodiments described above, the computer program executed by the control device 6 may be provided in a form recorded on a computer-readable portable recording medium such as a semiconductor memory, a magnetic recording medium, or an optical recording medium, or may be provided as a computer program product.

### DESCRIPTION OF REFERENCE SIGNS

- 6: control device (driving support control device)
- 100: vehicle

## Claims

1. A driving support control device (6) for a vehicle (100), configured to:
execute driving support for responding to driver abnormality in response to it being judged that a driver of the vehicle (100) is in an abnormal state in which it is difficult to continue driving of the vehicle (100);
suspend the driving support when a cancel operation is detected a plurality of times during the driving support; and
when the cancel operation includes a pedal operation of a pedal of the vehicle (100) and a predetermined operation other than the pedal operation, and the pedal operation is not detected even once as the cancel operation, not suspend the driving support even when the predetermined operation other than the pedal operation is detected the plurality of times.

2. The driving support control device (6) according to claim 1, configured to, when detecting an operation of releasing the pedal as the pedal operation, detect only an operation of releasing the pedal that was depressed at a start of the driving support, as the cancel operation.

3. The driving support control device (6) according to claim 1 or 2, configured to judge that the cancel operation has been performed once and increase a detection number of the cancel operation by one, each time either a steering operation of the vehicle (100) or the pedal operations is performed once.

4. The driving support control device (6) according to any one of claims 1 to 3, configured to return the detection number of the cancel operation to zero at a predetermined timing during the driving support.

5. The driving support control device (6) according to claim 4, wherein the driving support includes:
notification control being started after it is judged that the driver is in the abnormal state, and issuing a notification to the driver; and
deceleration and stop control being started after a predetermined time has elapsed since the notification started, and decelerating the vehicle (100) and maintaining the vehicle (100) in a stopped state, and
the predetermined timing is a time when a predetermined time has elapsed since the notification started, a time when a predetermined distance has been traveled since the notification started, a time when the notification control is ended, or a time when the deceleration and stop control is started.

6. A driving support method for a vehicle (100), including:
executing driving support for responding to driver abnormality in response to it being judged that a driver of the vehicle (100) is in an abnormal state in which it is difficult to continue driving of the vehicle (100);
suspending the driving support when a cancel operation is detected a plurality of times during the driving support; and
when the cancel operation includes a pedal operation of a pedal of the vehicle (100) and a predetermined operation other than the pedal operation, and the pedal operation is not detected even once as the cancel operation, not suspending the driving support even when the predetermined operation other than the pedal operation is detected the plurality of times.

7. A computer program which causes a computer to execute processing of:
executing driving support for responding to driver abnormality in response to it being judged that a driver of the vehicle (100) is in an abnormal state in which it is difficult to continue driving of the vehicle (100);
suspending the driving support when a cancel operation is detected a plurality of times during the driving support; and
when the cancel operation includes a pedal operation of a pedal of the vehicle (100), and a predetermined operation other than the pedal operation, and the pedal operation is not detected even once as the cancel operation, not suspending the driving support even when the predetermined operation other than the pedal operation is detected the plurality of times.
